# EUROPEAN PATENT APPLICATION

(11) **EP 4 609 854 A1**
(43) Date of publication of application: **03.09.2025**
(21) Application number: 23882997.2
(22) Date of filing: 19.10.2023
(51) Int. Cl.: A61K 9/00, A61K 9/14, A61K 31/465

(54) **LOW-DOSE NICOTINE DRY POWDER INHALATION COMPOSITION**

(30) Priority: 27.10.2022 KR 20220140558; 06.10.2023 KR 20230133754
(71) Applicant: KT&G Corporation, Daedeok-gu Daejeon 34337 (KR)
(72) Inventor: JUNG, Yongmi, Daejeon 34128 (KR); KIM, Moonwon, Daejeon 34128 (KR); SEO, Man Seok, Daejeon 34128 (KR); SHIN, Jun Won, Daejeon 34128 (KR)
(74) Representative: Ter Meer Steinmeister & Partner
(86) International application number: PCT/KR2023/016228
(87) International publication number: WO 2024/090890

(57) **Abstract**

The present invention provides: a nicotine dry powder inhalation composition; and a method for producing same. The nicotine dry powder inhalation composition comprises nicotine powder and flavor powder, the composition being characterized in that: the nicotine powder includes a nicotine or nicotine salt, an amino acid, and an excipient; the flavor powder includes a flavor and an excipient; and the nicotine content is less than 0.5 wt% with respect to the total dry powder composition.

## Description

### TECHNICAL FIELD

The present disclosure relates to a low-dose nicotine dry powder inhalation composition.

### BACKGROUND ART

Nicotine is delivered into the body by various methods, such as smoking combustible tobacco such as cigarettes, cigars, or pipes, using a method of generating an aerosol by heating an aerosol-generating substance in a cigarette, or directly inhaling a nicotine powder or the like.

Among these, the method of directly inhaling the nicotine powder uses a dry powder inhaler (DPI) to deliver the dry powder directly into the user's respiratory tract by inhalation. In addition to nicotine, the dry powder is also used to contain various effective ingredients (drugs) for the purpose of improving diseases, and the like.

However, since a nicotine dry powder for inhalation of the related art has a nicotine content of 1 to 10 wt%, there is an aspect of inducing coughing when inhaled, and the coughing induced by inhalation causes the discharge of the inhaled active components, etc., which prevents the designed function from being fully achieved.

### DISCLOSURE OF THE INVENTION

### TECHNICAL GOALS

One object of the present disclosure is to solve the above problems, and to provide a nicotine dry powder composition for inhalation, capable of suppressing coughing caused by a high nicotine content when inhaling a nicotine dry powder, and sufficiently and effectively obtaining a designed nicotine function through appropriate adjustment of a nicotine powder and a flavoring powder, and a method of manufacturing the same.

However, goals to be achieved are not limited to those described above, and other goals not mentioned above are clearly understood by one of ordinary skill in the art from the following description.

### TECHNICAL SOLUTIONS

According to one embodiment of the present disclosure, there is provided a nicotine dry powder composition for inhalation including a nicotine powder, and a flavoring powder,
wherein the nicotine powder includes nicotine or a nicotine salt, amino acid, and an excipient,
the flavoring powder includes a flavoring agent and an excipient, and
a content of the nicotine is less than 0.5 wt% based on a total weight of the dry powder composition.

### EFFECTS OF THE INVENTION

When a nicotine dry powder composition for inhalation according to an aspect of the present disclosure is used, coughing is suppressed to suppress the discharge of inhaled active components, so that a user may sufficiently and effectively obtain a designed nicotine and active component function, thereby maximizing the satisfaction of nicotine or the like.

### BRIEF DESCRIPTION OF DRAWINGS

FIG. 1 is a diagram illustrating spray drying process and conditions according to one embodiment of the present disclosure.
FIG. 2 diagram showing jet milling process and conditions of one embodiment of the disclosure.
FIG. 3 is a graph showing results of a test evaluation of a next generation impactor (NGI), which is a standard device for evaluating pharmaceutical inhalers, for cases where a nicotine content is 0.99 wt%, 0.74 wt%, and 0.5 wt%.
FIG. 4 is a diagram illustrating a result of measuring an amount of a nicotine dry powder composition transferred per puff according to one embodiment of the present disclosure.

### BEST MODE FOR CARRYING OUT THE INVENTION

Hereinafter, embodiments will be described in detail with reference to the accompanying drawings. However, various alterations and modifications may be made to the embodiments. Here, the embodiments are not construed as limited to the disclosure. The embodiments should be understood to include all modifications, equivalents, and substitutions within the scope and spirit of the present disclosure.

The terminology used herein is for the purpose of describing particular embodiments only and is not to be limiting of the embodiments. As used herein, the singular form is intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, the same or similar elements are denoted by the same reference numerals even though they are depicted in different drawings, and redundant descriptions thereof will be omitted. In the following description of the embodiments, a detailed description of known functions and configurations incorporated herein will be omitted when the same may make the subject matter of the embodiments disclosed in the present specification rather unclear.

In addition, the terms first, second, A, B, (a), and (b) may be used to describe constituent elements of the embodiments. These terms are used only for the purpose of discriminating one component from another component, and the nature, the sequences, or the orders of the components are not limited by the terms.

A component, which has the same common function as a component included in any one embodiment, will be described by using the same name in other embodiments. Unless otherwise mentioned, the descriptions of one embodiment may be applicable to other embodiments and thus, repeated descriptions will be omitted for conciseness.

The term "dry powder inhaler (DPI)" used herein refers to a device that delivers substances produced by a reaction between a nicotine-related substance and an organic acid substance into the user's lungs through the user's oral cavity.

According to one embodiment of the present disclosure, there is provided a nicotine dry powder composition for inhalation including a nicotine powder, and a flavoring powder,
wherein the nicotine powder includes nicotine or a nicotine salt, amino acid, and an excipient,
the flavoring powder includes a flavoring agent and an excipient, and
a content of the nicotine is less than 0.5 wt% based on a total weight of the dry powder composition.

First, the nicotine powder will be described in detail.

The nicotine powder according to one embodiment of the present disclosure is a component forming the nicotine dry powder composition for inhalation and corresponds to nicotine or a nicotine salt.

The nicotine salt here may be present in the form of a salt of nicotine and organic acid, and the organic acid may correspond to, but is not limited to, lactic acid, tartaric acid, pyruvic acid, benzoic acid, citric acid, and/or salicylic acid.

When forming the nicotine salt with the organic acid as described above, the nicotine salt may be in the form of, for example, nicotine lactate, nicotine tartrate, nicotine pyruvate, or nicotine salicylate. Among these, since the nicotine powder is used as the nicotine dry powder composition for human inhalation, the organic acid is desirably lactic acid, which has the advantage of being a metabolic substance in the body, non-toxic, harmless to the human body, and easy to decompose and excrete even after being absorbed by the human body, and does not have a negative effect on sensory characteristics and is not particularly unfavorable. Accordingly, it is desirable that the nicotine salt is nicotine lactate.

The nicotine powder may contain amino acid and an excipient, in addition to the nicotine or the nicotine salt.

The type of amino acid is not particularly limited, but may include one or more of leucine, lysine, valine, arginine, threonine, phenylalanine, glycine and/or methionine, and the amino acid may desirably include leucine.

As described above, when leucine is included as the amino acid, unlike in a case of other amino acids, since leucine has hydrophobicity, it is distributed on an outer surface of a powder during powder formation, thereby improving the flowability of a finally formed nicotine dry powder composition, and improving the transfer amount.

In addition, it is desirable that the content of amino acid included at this time is 5 wt% or less based on a total content of the dry powder. By controlling the content of amino acid to 5 wt% or less as described above, a particle size and particle size distribution of the finally formed nicotine dry powder may be improved, and it also corresponds to the FDA approval for the content of leucine.

The type of excipient included in the nicotine powder may include sugar and/or sugar alcohol, and the type of sugar may include sucrose, trehalose, dextrose, glucose, maltose, and the like. Furthermore, the type of sugar alcohol may include mannitol, sorbitol, galactitol, maltitol, xylitol, lactitol, and the like.

Among these, it is desirable that the excipient include mannitol, which has an advantage in formulation through spray drying. Specifically, the mannitol is water-soluble, stable in moisture before and after formulation, has no particularly negative sensory characteristics other than a slight sweetness, and has no particular side effects, and accordingly, it is particularly advantageous for use as a carrier material for delivering nicotine. In addition, the mannitol has the advantage of being able to bind to sputum (phlegm) in the bronchial tubes to easily discharge sputum (phlegm) when absorbed into the bronchial tubes.

Meanwhile, the nicotine dry powder composition for inhalation according to one embodiment of the present invention includes a flavoring powder as another component, and the flavoring powder will be described in detail below.

The flavoring powder may include a flavoring agent and an excipient. The flavoring agent refers to a flavor commonly used in a smoking article and the like, and may include, for example, rosemary, eucalyptol, licorice, sucrose, fructose syrup, isosweet, cocoa, lavender, cinnamon, cardamom, celery, fenugreek, cascarilla, sandalwood, bergamot, geranium, honey essence, rose oil, vanilla, lemon oil, orange oil, mint oil, mandarin oil, catechin, grapefruit, caraway, cognac, jasmine, chamomile, menthol, cinnamon, ylang-ylang, sage, spearmint, ginger, coriander, and coffee, but is not limited thereto.

Furthermore, by containing a flavoring agent in the flavoring powder, it is possible to not only evoke a sense of flavor for the user, but also exhibit a cough suppressing function, such as expanding the bronchial tubes, in addition to its role as a flavoring agent, optionally depending on the flavoring agent. In this respect, it is desirable that the flavoring agent include menthol.

In addition, as in the nicotine powder described above, the flavoring powder may also contain the excipient, and the type of excipient may include sugar including sucrose, trehalose, dextrose, glucose, or maltose and/or sugar alcohol including mannitol, sorbitol, galactitol, maltitol, xylitol, or lactitol as described in detail above. Among them, it is desirable to use mannitol as the excipient.

The nicotine dry powder composition for inhalation according to one embodiment of the present disclosure may contain the nicotine powder and the flavoring powder at a weight ratio of 9:1 to 5:5, desirably at a weight ratio of 8:2 to 6:4.

By adjusting the nicotine powder and the flavoring powder within the above range, the aerodynamic delivery characteristics of the powder may be adjusted, particularly, the particle size, the particle distribution, and the transfer characteristics of the powder are good, and thus, the user may satisfy both the functions of nicotine and flavor. Specifically, when a weight ratio of the nicotine powder to the flavoring powder exceeds 9:1 (e.g., 10:1, etc.), the impact on the upper bronchial tubes may be reduced, and when the weight ratio of the nicotine powder to the flavoring powder is less than 5:5 (e.g., 4:6, etc.), the particles tend to become larger, making it difficult for the flavoring particles to pull and deliver the nicotine particles to the inside of the bronchial tubes.

In addition, in terms of suppressing cough induction for the user, it is desirable that the content of nicotine finally included in the nicotine dry powder composition for inhalation is less than 0.5%, and when the nicotine content is higher than this, the efficiency of use (inhalation) of nicotine inhaled compared to the amount of nicotine provided may decrease due to the cough induction.

Meanwhile, the nicotine dry powder composition for inhalation according to one embodiment of the present invention may further contain a cough suppressant component, in addition to the nicotine powder and the flavoring powder described in detail above, and such cough suppressant component is not particularly limited, but may include menthol, mint, saccharin, benzocaine, and the like.

As described above, even when the nicotine dry powder composition for inhalation includes the cough suppressant component separately, in order to exhibit the effect according to one embodiment of the present disclosure, the nicotine content, the amino acid content, the weight ratio of the nicotine powder to the flavoring powder, and the like need to satisfy the desirable ranges described above.

According to one embodiment of the present invention, there is provided a method of manufacturing a nicotine dry powder composition for inhalation, the method including:
a step S1 of manufacturing a nicotine powder including nicotine or a nicotine salt, amino acid, and an excipient;
a step S2 of manufacturing a flavoring powder including a flavoring agent and an excipient; and
a step S3 of mixing the nicotine powder and the flavoring powder at a weight ratio of 8:2 to 6:4,
wherein a content of the nicotine is less than 0.5 wt% based on a total weight of the dry powder composition.

The composition and type of nicotine powder and flavoring powder manufactured at each step of the manufacturing method may be considered to be substantially the same as described in detail above, and each step of the manufacturing method will be described in detail below.

The step S1 is a step of manufacturing a nicotine powder including nicotine or a nicotine salt, amino acid, and an excipient, specifically through a spray drying process.

In spray drying, water is used as a solvent, this is mixed with a substance forming the nicotine powder, then, the spray drying is performed at an inlet temperature (inlet temp.) of about 100°C at a flow rate of about 2 to 7 mL/min to form a nicotine powder.

Then, the step S2 is a step of manufacturing a flavoring powder including a flavoring agent and an excipient, specifically through a jet milling process.

When performing the jet milling, it is desirable to lower the temperature so that the temperature does not rise. This is because the flavoring agent such as menthol easily evaporate at a high temperature, which results in a decrease in the content included in the powder or a negative effect on the transfer amount. Therefore, it is desirable to manufacture the flavoring powder in a separate step through low-temperature jet milling, unlike the nicotine powder, which is spray-dried at a high temperature.

Thereafter, in the step S3, the manufactured nicotine powder and flavoring powder may be mixed (post-mixed) at a weight ratio of 8:2 to 6:4, through which the components such as nicotine contained in the nicotine powder and the flavoring powder may be evenly mixed without loss of the components, and flavoring particles having a relatively large particle size serve as a carrier for delivering the nicotine particles, thereby showing excellent results in terms of transition characteristics when inhaled by the user.

Hereinafter, the configuration of the present disclosure and effects thereof will be described in more detail through examples and comparative examples. However, the examples are merely intended for the purpose of describing the disclosure in more detail, and thus, the scope of the disclosure is not limited to the examples.

### Examples

### 1. Manufacturing Example: Manufacturing of nicotine dry powder composition for inhalation

### 1) Manufacturing of nicotine powder - Spray drying

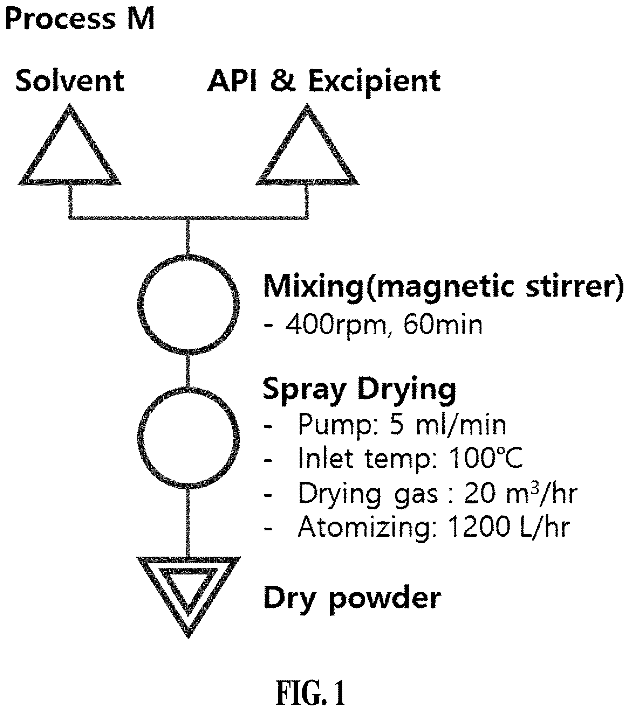

Using water as a solvent, nicotine, lactic acid, mannitol, and leucine were mixed at the composition ratios shown in Table 1 below, and a nicotine powder was manufactured according to spray drying process shown in FIG. 1 and process conditions shown in Table 2.

**[Table 1]**

| Components for forming Nicotine powder | Composition ratio |
|---|---|
| Nicotine | 0.83% |
| Lactic acid | 4.00% |
| Mannitol | 90.17% |
| Leucine | 5.00% |
| Total | 100.00% |

**[Table 2]**

| Device | Inlet T | Pump | Spray gas | Drying gas |
|---|---|---|---|---|
| Buchi-300 | 100°C | 5 ml/min | 1200 L/hr | 20 m³/hr |

| | | | | |
|---|---|---|---|---|
| * Step: solid content (within 2%, aqueous solution at room temperature) | | | | |

### 2) Manufacturing flavoring powder - Jet milling

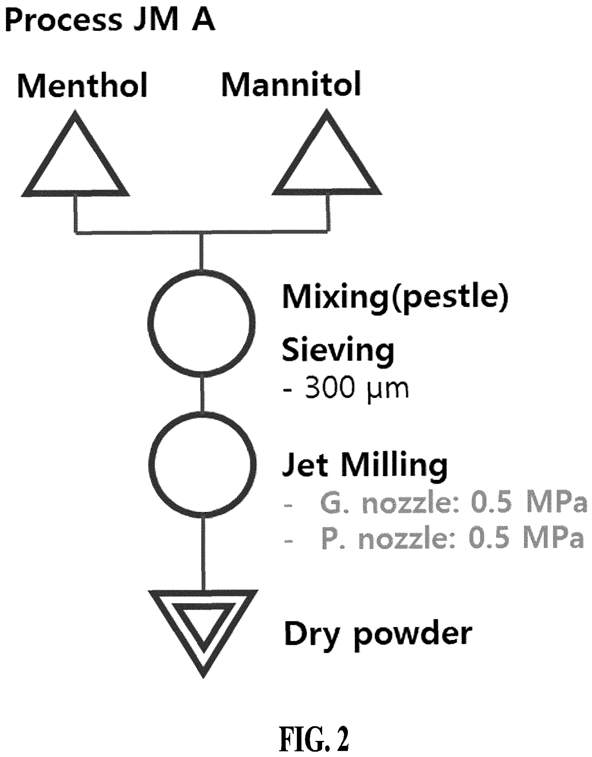

The jet milling process (conditions) shown in FIG. 2 was performed at the composition ratios shown in Table 3 to manufacture a flavoring powder. At this time, low-temperature jet milling was performed to prevent a temperature rise during the jet milling.

**[Table 3]**

| Components for forming flavoring powder | Composition ratio |
|---|---|
| Flavor (menthol) | 25.00% |
| Mannitol | 42.50% |
| Xylitol | 27.50% |
| Leucine | 5.00% |
| Total | 100.00% |

### 3) Manufacturing of final dry powder composition

The manufactured nicotine powder and flavoring powder were mixed at a weight ratio of 6:4 to manufacture a final nicotine dry powder composition, and composition ratios of the obtained nicotine dry powder composition are shown in Table 4 below.

**[Table 4]**

| Components for forming nicotine dry powder | Composition ratio |
|---|---|
| Nicotine | 0.50% (0.498%) |
| Lactic acid | 2.40% |
| Mannitol | 71.10% |
| Xylitol | 11.00% |
| Leucine | 5.00% |
| Flavor (menthol) | 10.00% |
| Total | 100.00% |

### 2. Test Example 1: Measurement of particle size and particle size distribution

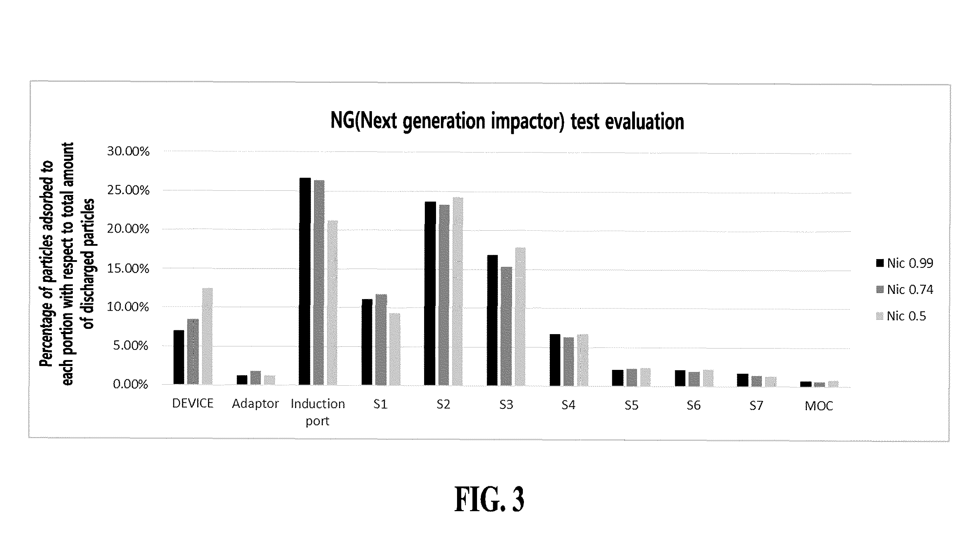

For the dry powder composition including 0.50 % of nicotine manufactured above, and each of dry powder compositions including 0.74% and 0.99% of nicotine, a test evaluation of an NGI, which is a standard device for evaluating pharmaceutical inhalers, was performed, and results thereof were shown in FIG. 3. In addition, using inhalatix program, results of a fine particle fraction (FPF) and a mass median aerodynamic diameter (MMAD) were derived, and the result for the dry powder composition including 0.50% of nicotine was shown in Table 5 below.

**[Table 5]**

| Device | MMAD | FPF |
|---|---|---|
| NGI (60 lpm) | 4.563*µ*m | 48.71% |

Meanwhile, the description in FIG. 3 is as follows.
Device: Inhaler device
Adaptor: Holder connecting an inhaler device and an induction port
Induction port: Device simulating the human respiratory system as an NGI component
S1 to S7: Impactor steps that captures aerosols by particle size in the NGI
MOC: Last part of the impactor
y-axis: Percentage of particles adsorbed to each portion with respect to a total amount of discharged particles (100%)

### 3. Test Example 2: Evaluation of transfer characteristics

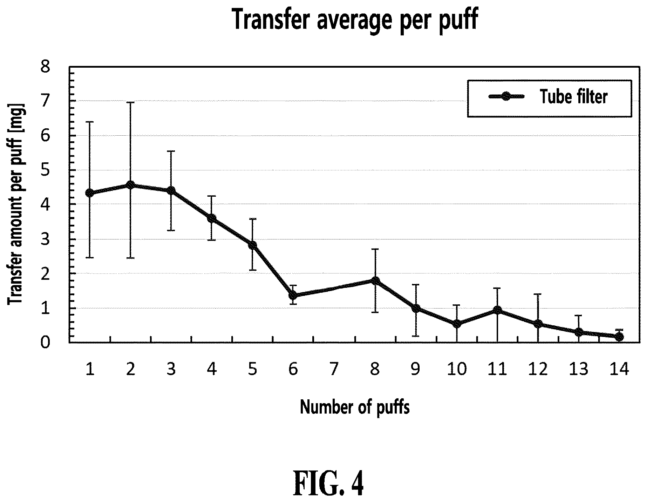

The nicotine dry powder composition manufactured above was applied to a tube filter, and the amount thereof transferred during 14 puffs was shown (FIG. 4).

When the total amount of transfer is 25 mg or more based on 30 mg of filling amount, it may be seen that the total amount of transfer is good. Thus, it may be seen that the nicotine dry powder composition according to one embodiment of the present disclosure has good transfer characteristics.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, and/or replaced or supplemented by other components or their equivalents.

Therefore, other implementations, other embodiments, and equivalents to the claims are also within the scope of the following claims.

## Claims

1. A nicotine dry powder composition for inhalation comprising:
a nicotine powder; and
a flavoring powder,
wherein the nicotine powder comprises nicotine or a nicotine salt, amino acid, and an excipient,
the flavoring powder comprises a flavoring agent and an excipient, and
a content of the nicotine is less than 0.5 wt% based on a total weight of the dry powder composition.

2. The nicotine dry powder composition for inhalation of claim 1, wherein a weight ratio of the nicotine powder to the flavoring powder is 8:2 to 6:4.

3. The nicotine dry powder composition for inhalation of claim 1, wherein the nicotine salt comprises nicotine, and a salt comprising one or more selected from the group consisting of lactic acid, tartaric acid, pyruvic acid, benzoic acid, citric acid, and salicylic acid.

4. The nicotine dry powder composition for inhalation of claim 1, wherein the nicotine salt comprises nicotine and lactic acid.

5. The nicotine dry powder composition for inhalation of claim 1, wherein the amino acid comprises one or more selected from the group consisting of leucine, lysine, valine, arginine, threonine, phenylalanine, glycine, and methionine.

6. The nicotine dry powder composition for inhalation of claim 1, wherein the amino acid comprises leucine.

7. The nicotine dry powder composition for inhalation of claim 1, wherein a content of the amino acid is 5 wt% or less based on the total weight of the dry powder composition.

8. The nicotine dry powder composition for inhalation of claim 1, wherein the excipient comprises one or more of sugar and sugar alcohol,
the sugar comprises one or more selected from the group consisting of lactose, sucrose, trehalose, dextrose, glucose, and maltose, and
the sugar alcohol comprises one or more selected from the group consisting of mannitol, sorbitol, galactitol, maltitol, xylitol, and lactitol.

9. The nicotine dry powder composition for inhalation of claim 1, wherein the excipient comprises mannitol.

10. The nicotine dry powder composition for inhalation of claim 1, wherein the flavoring agent comprises rosemary, eucalyptol, licorice, sucrose, fructose syrup, isosweet, cocoa, lavender, cinnamon, cardamom, celery, fenugreek, cascarilla, sandalwood, bergamot, geranium, honey essence, rose oil, vanilla, lemon oil, orange oil, mint oil, mandarin oil, catechin, grapefruit, caraway, cognac, jasmine, chamomile, menthol, cinnamon, ylang-ylang, sage, spearmint, ginger, coriander, and coffee.

11. The nicotine dry powder composition for inhalation of claim 1, further comprising:
a cough suppressant component.

12. The nicotine dry powder composition for inhalation of claim 11, wherein the cough suppressant component comprises one or more of menthol, mint, saccharin, and benzocaine.

13. A method of manufacturing a nicotine dry powder composition for inhalation, the method comprising:
a step S1 of manufacturing a nicotine powder comprising nicotine or a nicotine salt, amino acid, and an excipient;
a step S2 of manufacturing a flavoring powder comprising a flavoring agent and an excipient; and
a step S3 of mixing the nicotine powder and the flavoring powder at a weight ratio of 8:2 to 6:4,
wherein a content of the nicotine is less than 0.5 wt% based on a total weight of the dry powder composition.
